# EUROPÄISCHE PATENTSCHRIFT

(11) **EP 2 022 781 B1**
(45) Veröffentlichungstag und Bekanntmachung des Hinweises auf die Patenterteilung: **29.02.2012**
(21) Anmeldenummer: 08013824.1
(22) Anmeldetag: 01.08.2008
(51) Int. Cl.: C07D 207/277, C08F 26/00, C08G 63/66, C09K 8/00

(54) **1-Alkyl-5-oxo-pyrrolidin-3-carbonsäureester mit verbesserter biologischer Abbaubarkeit**
1-Alkyl-5-oxo-pyrrolidine-3-carboxylic acid esters with improved biodegradability
Esters d'acide 1-alkyl-5-oxo-pyrrolidine-3-carboxylique ayant une biodégrabilité améliorée

(30) Priorität: 06.08.2007 DE 102007037017
(43) Veröffentlichungstag der Anmeldung: 11.02.2009
(60) Teilanmeldung: 11008153.6
(73) Patentinhaber: Clariant Finance (BVI) Limited, Road Town, Tortola (VG)
(72) Erfinder: Leinweber, Dirk, 65779 Kelkheim (DE); Rösch, Alexander, 67578 Gimbsheim (DE); Feustel, Michael, 55278 Köngerheim (DE)
(74) Vertreter: Mikulecky, Klaus

(56) Entgegenhaltungen:
- WO-A-2006/084613
- WO-A-2007/054225
- DE-A1- 4 333 238
- DE-A1- 10 259 815
- DE-A1-102005 054 037
- DE-B3-102007 037 015
- US-A- 4 127 493

## Beschreibung

Die vorliegende Erfindung betrifft 1-Alkyl-5-oxo-pyrrolidin-3-carbonsäureester und ihre Verwendung als Gashydratinhibitoren.

Gashydrate sind kristalline Einschlussverbindungen von Gasmolekülen in Wasser, die sich unter bestimmten Temperatur- und Druckverhältnissen (niedrige Temperatur und hoher Druck) bilden. Hierbei bilden die Wassermoleküle Käfigstrukturen um die entsprechenden Gasmoleküle aus. Das aus den Wassermolekülen gebildete Gittergerüst ist thermodynamisch instabil und wird erst durch die Einbindung von Gastmolekülen stabilisiert. Diese eisähnlichen Verbindungen können in Abhängigkeit von Druck und Gaszusammensetzung auch über den Gefrierpunkt von Wasser (bis über 25 °C) hinaus existieren.

In der Erdöl- und Erdgasindustrie sind insbesondere die Gashydrate von großer Bedeutung, die sich aus Wasser und den Erdgasbestandteilen Methan, Ethan, Propan, Isobutan, n-Butan, Stickstoff, Kohlendioxid und Schwefelwasserstoff bilden. Insbesondere in der heutigen Erdgasförderung stellt die Existenz dieser Gashydrate ein großes Problem dar, besonders dann, wenn Nassgas oder Mehrphasengemische aus Wasser, Gas und Alkangemischen unter hohem Druck niedrigen Temperaturen ausgesetzt werden. Hier führt die Bildung der Gashydrate aufgrund ihrer Unlöslichkeit und kristallinen Struktur zur Blockierung verschiedenster Fördereinrichtungen, wie Pipelines, Ventilen oder Produktionseinrichtungen, in denen über längere Strecken bei niedrigeren Temperaturen Nassgas oder Mehrphasengemische transportiert werden, wie dies speziell in kälteren Regionen der Erde oder auf dem Meeresboden vorkommt.

Außerdem kann die Gashydratbildung auch beim Bohrvorgang zur Erschließung neuer Gas- oder Erdöllagerstätten bei entsprechenden Druck- und Temperaturverhältnissen zu Problemen führen, indem sich in den Bohrspülflüssigkeiten Gashydrate bilden.

Um solche Probleme zu vermeiden, kann die Gashydratbildung in Gaspipelines, beim Transport von Mehrphasengemischen oder in Bohrspülflüssigkeiten durch Einsatz von größeren Mengen (mehr als 10 Gew.-% bezüglich des Gewichts der Wasserphase) niederen Alkoholen, wie Methanol, Glykol, oder Diethylenglykol unterdrückt werden. Der Zusatz dieser Additive bewirkt, dass die thermodynamische Grenze der Gashydratbildung zu niedrigeren Temperaturen und höheren Drücken hin verlagert wird (thermodynamische Inhibierung). Durch den Zusatz dieser thermodynamischen Inhibitoren werden allerdings größere Sicherheitsprobleme (Flammpunkt und Toxizität der Alkohole), logistische Probleme (große Lagertanks, Recycling dieser Lösungsmittel) und dementsprechend hohe Kosten, speziell in der offshore-Förderung, verursacht.

Heute versucht man deshalb, thermodynamische Inhibitoren zu ersetzen, indem man bei Temperatur- und Druckbereichen, in denen sich Gashydrate bilden können, Additive in Mengen < 2 % zusetzt, die die Gashydratbildung entweder zeitlich hinauszögern (kinetische Inhibitoren) oder die Gashydratagglomerate klein und damit pumpbar halten, so dass diese durch die Pipeline transportiert werden können (sog. Agglomerat-Inhibitoren oder Antiagglomerants). Die dabei eingesetzten Inhibitoren behindern entweder die Keimbildung und/oder das Wachstum der Gashydratpartikel, oder modifizieren das Hydratwachstum derart, dass kleinere Hydratpartikel resultieren.

Als Gashydratinhibitoren wurden in der Patentliteratur neben den bekannten thermodynamischen Inhibitoren eine Vielzahl monomerer als auch polymerer Substanzklassen beschrieben, die kinetische Inhibitoren oder Antiagglomerants darstellen. Von besonderer Bedeutung sind hierbei Polymere mit Kohlenstoff-Backbone, die in den Seitengruppen sowohl cyclische (Pyrrolidon- oder Caprolactamreste) als auch acyclische Amidstrukturen enthalten.

So wird in WO-94/12761 ein Verfahren zur kinetischen Inhibierung der Gashydratbildung durch die Verwendung von Polyvinyllactamen mit einem Molekulargewicht von M_{W} > 40.000 D, und in WO-93/25798 wird ein solches Verfahren unter Verwendung von Polymeren und/oder Copolymeren des Vinylpyrrolidons mit einem Molekulargewicht von M_{W} > 5.000 bis 40.000 D offenbart.

EP-A-0 896 123 offenbart Gashydratinhibitoren, die Copolymere aus alkoxylierter Methacrylsäure ohne Alkyl-Endverschluss und cyclischen N-Vinylverbindungen enthalten können.

In US-5 244 878 wird ein Verfahren zum Verzögern der Bildung oder Vermindern der Bildungstendenz von Gashydraten beschrieben. Hierzu werden Polyole verwendet, die mit Fettsäuren oder Alkenylbernsteinsäureanhydriden verestert werden. Die hergestellten Verbindungen weisen keine Aminosäurefunktionen auf, die mit Clathraten (Käfigmolekülen) interagieren könnten.

Die beschriebenen Additive besitzen nur eingeschränkte Wirksamkeit als kinetische Gashydratinhibitoren und/oder Antiagglomerants, müssen mit Co-Additiven verwendet werden, oder sind nicht in ausreichender Menge oder nur zu hohen Preisen erhältlich.

Um Gashydratinhibitoren auch bei stärkerer Unterkühlung als derzeit möglich, d. h. weiter innerhalb der Hydratregion einsetzen zu können, bedarf es einer weiteren Wirkungssteigerung im Vergleich zu den Hydratinhibitoren des Standes der Technik. Zusätzlich sind in Bezug auf ihre biologische Abbaubarkeit verbesserte Produkte erwünscht.

Aufgabe der vorliegenden Erfindung war es also, Additive zu finden, die sowohl die Bildung von Gashydraten verlangsamen (kinetische Inhibitoren) als auch Gashydratagglomerate klein und pumpbar halten (Antiagglomerants), um so ein breites Anwendungsspektrum mit hohem Wirkpotential zu gewährleisten. Des Weiteren sollten sie in der Lage sein, die derzeit verwendeten thermodynamischen Inhibitoren (Methanol und Glykole), die beträchtliche Sicherheitsprobleme und Logistikprobleme verursachen, zu ersetzen.

Da derzeit verwendete Inhibitoren wie Polyvinylpyrrolidon und Polyvinylcaprolactam nur eine mäßige biologische Abbaubarkeit aufweisen, sollten die erfindungsgemäßen Verbindungen zudem eine verbesserte biologische Abbaubarkeit aufweisen.

Im Stand der Technik sind 1-Alkyl-5-oxo-pyrrolidin-3-carbonsäurederivate bekannt, die nicht als Gashydratinhibitoren sondern in anderen Gebieten eingesetzt werden.

So offenbart GB-A-1 323 061 N-substituierte 5-Oxo-pyrrolidin-3-carbonsäuren und deren Verwendung in Funktionsflüssigkeiten, insbesondere Hydraulikflüssigkeiten.

US-3 224 975 offenbart 1-Alkyl-5-oxo-pyrrolidin-3-carbonsäuren, die als Korrosionsschutz-Additive in Schmiermitteln eingesetzt werden.

US-4 127 493 offenbart Polyester, die durch Umsetzung von N-substituierten 5-oxo-pyrrolidin-3-carbonsäuren oder -estern mit Alkenylbernsteinsäure oder Alkenylbernsteinsäureanhydrid hergestellt werden, und deren Verwendung als öllösliche Additive in Schmiermitteln.

EP-A-0 069 512 offenbart N-substituierte 5-oxo-pyrrolidin-3-carbonsäuresalze und deren Verwendung als Befeuchtungsmittel.

Wie nun überraschenderweise gefunden wurde, eignen sich sowohl wasserlösliche als auch öllösliche 1-Alkyl-5-oxo-pyrrolidin-3-carbonsäureester als Gashydratinhibitoren. Diese Ester können je nach Struktur sowohl die Keimbildung und das Wachstum von Gashydraten verzögern (kinetische Gashydratinhibitoren) als auch die Agglomeration von Gashydraten unterdrücken (Antiagglomerants). Zudem weisen die erfindungs-gemäßen Verbindungen eine deutlich verbesserte biologische Abbaubarkeit auf.

Gegenstand der vorliegenden Erfindung sind Verbindungen der Formel 1 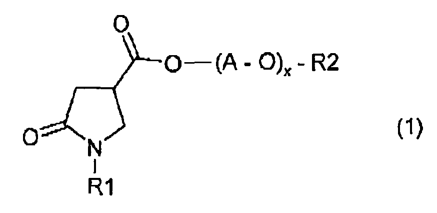 worin
- A: eine C₂- bis C₄-Alkylengruppe
- x: eine Zahl von 1 bis 100,
- y: eine Zahl von 0 bis 100 bedeuten,
- R1: für C₁-C₃₀-Alkyl, C₂-C₃₀₋Alkenyl, C₇-C₃₀-Alkylaryl steht,
- R2: für einen organischen Rest der Formel 3 steht 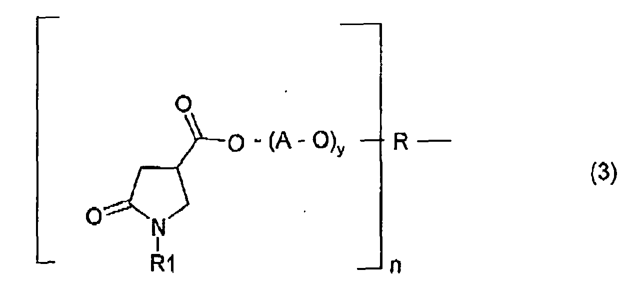 worin n für eine Zahl von 1 bis 100 steht, und R entweder
(1) durch formale Abstraktion der Wasserstoffatome der OH-Gruppen von Ethylenglykol, Propylenglykol, Glycerin, Diglycerin, Triglycerin, Tetraglycerin, Polyglycerin, Trimethylolpropan, Pentaerythrit, Dipentaerythrit, Tripentaerythrit, Sorbitol, Sorbitan, Diethylenglykol, Triethylenglykol, Dlpropylenglykol, Tripropylenglykol und Tris(hydroxymethyl)aminomethan gebildet wird,
   oder
(2) für einen Alkylen- oder Polyalkylenoxid-Rest mit 1 bis 30 Kohlenstoffatomen steht.

Ein weiterer Gegenstand der Erfindung ist die Verwendung von Verbindungen der Formel 1 in Mengen von 0,01 bis 2 Gew.-% (bezogen auf das Gewicht der wässrigen Phase) zur Verhinderung der Bildung von Gashydraten in wässrigen Phasen, die mit einer gasförmigen, flüssigen oder festen organischen Phase in Verbindung stehen.

Ein weiterer Gegenstand der Erfindung ist ein Verfahren zur Inhibierung der Bildung von Gashydraten, indem man einer mit einer gasförmigen, flüssigen oder festen organischen Phase in Verbindung stehenden wässrigen Phase, in der die Gashydratbildung verhindert werden soll mindestens eine Verbindung der Formel 1 in Mengen von 0,01 bis 2 Gew.-% (bezogen auf das Gewicht der wässrigen Phase) zusetzt.

R2 steht für einen organischen Rest derFormel 3 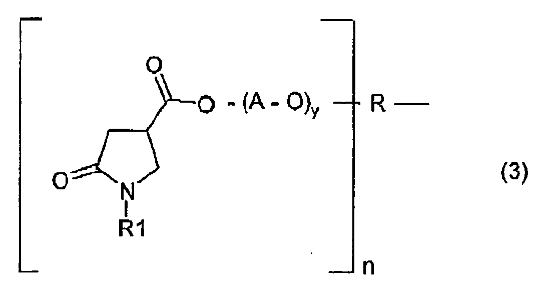 worin n für 1 - 100 steht, und R für einen Alkylen- oder Polyalkylenoxid-Rest mit 1 bis 30 Kohlenstoffatomen steht, oder

R entsteht durch formale Abstraktion der Wasserstoffatome der OH-Gruppen von Ethylenglykol, Propylenglykol, Glycerin, Diglycerin, Triglycerin, Tetraglycerin, Polyglycerin, Trimethylolpropan, Pentaerythrit, Dipentaerythrit, Tripentaerythrit, Sorbitol, Sorbitan, Diethylenglykol, Triethylenglykol, Dipropylenglykol, Tripropylenglykol und Tris(hydroxymethyl)aminomethan. Es können alle oder nur ein Teil der Wasserstoffatome der Polyole bei der Bildung von R abstrahiert werden. Bevorzugt ist es, wenn 2, 3, 4, 5, oder 6 Wasserstoffatome der OH-Gruppen des Polyols abstrahiert werden, d.h. Reste der Formel 2 tragen.

Ein weiter Gegenstand der Erfindung ist eine Verbindung der Formel 4 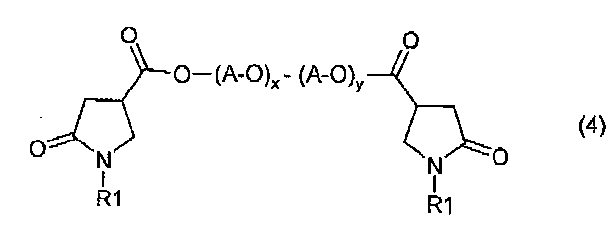 worin y in diesem Fall für eine Zahl von 1 bis 100, steht.

Erfindungsgemäß können auch Mischungen von mit unterschiedlichen Resten R1 substituierten 1-Alkyl-5-oxo-pyrrolidin-3-carbonsäuren zur Herstellung der 1-Alkyl-5-oxo-pyrrolidin-3-carbonsäureester eingesetzt werden.

A steht vorzugsweise für Ethylenreste oder für Mischungen aus Ethylen- und Propylenresten.

In der durch (A-O)ₓ oder (A-O)_{y} wiedergegebenen Alkoxykette bedeutet A vorzugsweise einen Ethylen- oder Propylenrest, insbesondere einen Ethylenrest. x und y bedeuten vorzugsweise unabhängig voneinander eine Zahl zwischen 1 und 80, insbesondere zwischen 2 und 70, speziell zwischen 3 und 50. Bei der Alkoxykette kann es sich um eine Blockpolymerkette handeln, die alternierende Blöcke verschiedener Alkoxyeinheiten, vorzugsweise Ethoxy- und Propoxyeinheiten, aufweist. Es kann sich dabei auch um eine Kette mit statistischer Abfolge der Alkoxyeinheiten oder um ein Homopolymer handeln.

In einer bevorzugten Ausführungsform steht -(A-O)ₓ- oder -(A-O)_{y}- für eine Alkoxykette der Formel

- (CH₂ - CH₂ - O)ₐ -- (CH₂ - CH(CH₃) - O)_{b} -- (CH₂ - CH₂ - O)_{c} -

worin
- a: eine Zahl von 1 bis 100, vorzugsweise 5 bis 80
- b: eine Zahl von 0 bis 100, vorzugsweise 5 bis 100
- c: eine Zahl von 1 bis 100, vorzugsweise 5 bis 80 bedeuten.

Allen Ausführungsformen gemein ist, dass vorzugsweise mindestens 50 mol-% der Reste (A-O) Ethoxyreste sind, insbesondere sind 60 bis 100 mol-% Ethoxyreste.

Beispiele erfindungsgemäßer Verbindungen sind in den folgenden Formeln 5 und 6 angegeben: 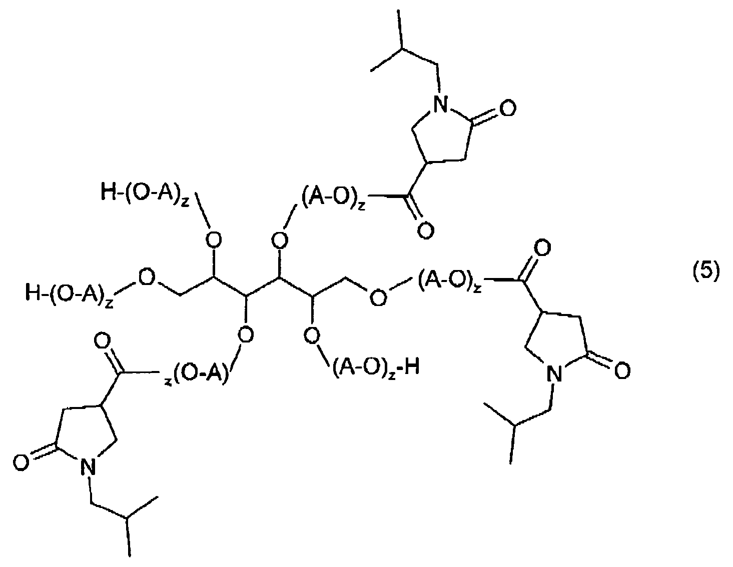 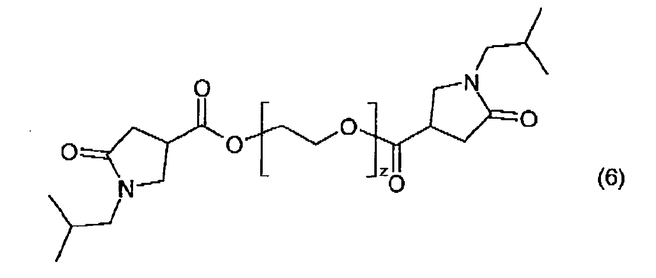 worin z die für x und y angegebenen Werte annehmen kann.

Die erfindungsgemäßen 1-Alkyl-5-oxo-pyrrolidin-3-carbonsäureester sind durch Veresterung von 1-Alkyl-5-oxo-pyrrolidin-3-carbonsäure mit mindestens einem Alkohol der Formel HO-(A-O)ₓ-R-(O-A)_{y}-OH darstellbar. Die Herstellung der 1-Alkyl-5-oxo-pyrrolidin-3-carbonsäuren erfolgt wie im Stand der Technik ausführlich beschrieben durch Umsetzung von Itaconsäure mit primären Aminen und kann wie in EP-A-0 069 512, US-3 224 975 und US-4 127 493 beschrieben durchgeführt werden.

Die Herstellung der erfindungsgemäßen Ester ist im Stand der Technik bekannt und erfolgt durch unkatalysierte oder sauer katalysierte Kondensation der Carbonsäure mit dem entsprechenden Alkohol. Die Reaktionstemperatur liegt im Allgemeinen zwischen 100 und 300°C, vorzugsweise bei 170 bis 250°C. Das bei der Veresterung zur Anwendung kommende molare Verhältnis von OH-Gruppen im Alkohol zur 1-Alkyl-5-oxo-pyrrolidin-3-carbonsäure liegt vorzugsweise zwischen 1 : 0,3 und 1 : 1, insbesondere zwischen 1 : 0,5 und 1 : 1.

Die Reaktion kann bei Atmosphärendruck oder vermindertem Druck durchgeführt werden. Als katalysierende Säuren sind beispielsweise HCl, H₂SO₄, Sulfonsäuren, H₃PO₄ oder saure Ionentauscher zu nennen, die in Mengen von 0,1 bis 5 Gew.-%, bezogen auf das Gewicht des Reaktionsgemisches, verwendet werden. Die Veresterung nimmt im Allgemeinen 3 bis 30 Stunden in Anspruch.

Die erfindungsgemäßen 1-Alkyl-5-oxo-pyrrolidin-3-carbonsäureester können alleine oder in Kombination mit anderen bekannten Gashydratinhibitoren eingesetzt werden. Im Allgemeinen wird man dem zur Hydratbildung neigenden System soviel des erfindungsgemäßen 1-Alkyl-5-oxo-pyrrolidin-3-carbonsäureesters zusetzen, dass man unter den gegebenen Druck- und Temperaturbedingungen eine ausreichende Inhibierung erhält. Die erfindungsgemäßen 1-Alkyl-5-oxo-pyrrolidin-3-carbonsäureester werden vorzugsweise in Mengen zwischen 0,02 bis 2 Gew.-% (bezogen auf das Gewicht der wässrigen Phase) verwendet. Werden die erfindungsgemäßen 1-Alkyl-5-oxo-pyrrolidin-3-carbonsäureester in Mischung mit anderen Gashydratinhibitoren verwendet, so beträgt die Konzentration der Mischung 0,01 bis 2 bzw. 0,02 bis 1 Gew.-% in der wässrigen Phase.

Die 1-Alkyl-5-oxo-pyrrolidin-3-carbonsäureester werden vorzugsweise zur Anwendung als Gashydratinhibitoren in Wasser oder in wassermischbaren (bevorzugt alkoholischen) Lösungsmitteln wie beispielsweise Methanol, Ethanol, Propanol, Butanol, Ethylenglykol, 1,2-Propylenglykol, 1,3-Propylenglykol, Glycerin, Diethylenglykol, Triethylenglykol, N-Methylpyrrolidon sowie oxethylierten Monoalkoholen, wie Butylglykol, Isobutylglykol, Butyldiglykol gelöst. Öllösliche 1-Alkyl-5-oxo-pyrrolidin-3-carbonsäureester werden vorzugsweise zur Anwendung als Gashydratinhibitoren in unpolareren Lösungsmitteln wie C₃-C₈-Ketonen, beispielsweise Diisobutylketon, Methylisobutylketon, Cyclohexanon, oder C₅-C₁₂-Alkoholen, beispielsweise 2-Ethylhexanol, gelöst.

### Beispiele

### Herstellung der 1-Alkyl-5-oxo-pyrrolidin-3-carbonsäureester

### Beispiel 1

### Herstellung von Triethylenglykol-di(1-methyl-5-oxo-pyrrolidin-3-carboxylat)

In einem 500 ml Vierhalskolben mit Rührer, Thermometer, Stickstoffspülung sowie Destillationsbrücke wurden 143 g 1-Methyl-5-oxo-pyrrolidin-3-carbonsäure, 75 g Triethylenglykol sowie 1,0 g p-Toluolsulfonsäure vermischt und auf 200 °C erhitzt. Innerhalb von 8 h bei 200 °C wurden ca. 18 ml Wasser abdestilliert. Dabei wurden ca. 201 g Triethylenglykol-di(1-methyl-5-oxo-pyrrolidin-3-carboxylat) mit einer Verseifungszahl von 268 mg KOH/g erhalten.

### Beispiel 2

### Herstellung von (Trimethylolpropan + 9 EO)-tri(1-methyl-5-oxo-pyrrolidin-3-carboxylat)

In einem 500 ml Vierhalskolben mit Rührer, Thermometer, Stickstoffspülung sowie Destillationsbrücke wurden 143 g 1-Methyl-5-oxo-pyrrolidin-3-carbonsäure, 177 g Trimethylolpropan + 9 EO sowie 1,5 g p-Toluolsulfonsäure vermischt und auf 200 °C erhitzt. Innerhalb von 15 h bei 200 °C wurden ca. 18 ml Wasser abdestilliert. Dabei wurden ca. 304 g (Trimethylolpropan + 9 EO)-tri(1-methyl-5-oxo-pyrrolidin-3-carboxylat) mit einer Verseifungszahl von 186 mg KOH/g erhalten.

### Beispiel 3

### Herstellung von (Sorbitol + 18 EO)-tri(1-methyl-5-oxo-pyrrolidin-3-carboxylat)

In einem 1000 ml Vierhalskolben mit Rührer, Thermometer, Stickstoffspülung sowie Destillationsbrücke wurden 143 g 1-Methyl-5-oxo-pyrrolidin-3-carbonsäure, 325 g Sorbitol + 18 EO sowie 2,0 g p-Toluolsulfonsäure vermischt und auf 200 °C erhitzt. Innerhalb von 20 h bei 200 °C wurden ca. 18 ml Wasser abdestilliert. Dabei wurden ca. 451 g (Sorbitol + 18 EO)-tri(1-methyl-5-oxo-pyrrolidin-3-carboxylat) mit einer Verseifungszahl von 125 mg KOH/g erhalten.

### Beispiel 4

### Herstellung von (Sorbitol + 30 EO)-hexa(1-methyl-5-oxo-pyrrolidin-3-carboxylat)

In einem 500 ml Vierhalskolben mit Rührer, Thermometer, Stickstoffspülung sowie Destillationsbrücke wurden 143 g 1-Methyl-5-oxo-pyrrolidin-3-carbonsäure, 250 g Sorbitol + 30 EO sowie 2,0 g p-Toluolsulfonsäure vermischt und auf 200 °C erhitzt. Innerhalb von 20 h bei 200 °C wurden ca. 18 ml Wasser abdestilliert. Dabei wurden ca. 376 g (Sorbitol + 30 EO)-hexa(1-methyl-5-oxo-pyrrolidin-3-carboxylat) mit einer Verseifungszahl von 149 mg KOH/g erhalten.

### Beispiel 5

### Herstellung von (Dekaglycerin + 30 EO)-poly(1-methyl-5-oxo-pyrrolidin-3-carboxylat)

In einem 1000 ml Vierhalskolben mit Rührer, Thermometer, Stickstoffspülung sowie Destillationsbrücke wurden 143 g 1-Methyl-5-oxo-pyrrolidin-3-carbonsäure, 390 g Dekaglycerin + 30 EO sowie 2,5 g p-Toluolsulfonsäure vermischt und auf 200 °C erhitzt. Innerhalb von 30 h bei 200 °C wurden ca. 18 ml Wasser abdestilliert. Dabei wurden ca. 516 g (Dekaglycerin + 30 EO)-poly(1-methyl-5-oxo-pyrrolidin-3-carboxylat) mit einer Verseifungszahl von 104 mg KOH/g erhalten.

### Referenz-Beispiel 6

### Herstellung von (Polypropylenglykol 400 + 10 EO)-di(1-methyl-5-oxo-pyrrolidin-3-carboxylat)

In einem 1000 ml Vierhalskolben mit Rührer, Thermometer, Stickstoffspülung sowie Destillationsbrücke wurden 143 g 1-Methyl-5-oxo-pyrrolidin-3-carbonsäure, 420 g Polypropylenglykol 400 + 10 EO sowie 2,5 g p-Toluolsulfonsäure vermischt und auf 200 °C erhitzt. Innerhalb von 18 h bei 200 °C wurden ca. 18 ml Wasser abdestilliert. Dabei wurden ca. 546 g (Polypropylenglykol 400 + 10 EO)-di(1-methyl-5-oxo-pyrrolidin-3-carboxylat) mit einer Verseifungszahl von 103 mg KOH/g erhalten.

### Referenz-Beispiel 7

### Herstellung von (Polypropylenglykol 600)-di(1-methyl-5-oxo-pyrrolidin-3-carboxylat)

In einem 1000 ml Vierhalskolben mit Rührer, Thermometer, Stickstoffspülung sowie Destillationsbrücke wurden 143 g 1-Methyl-5-oxo-pyrrolidin-3-carbonsäure, 300 g Polypropylenglykol 600 sowie 2,0 g p-Toluolsulfonsäure vermischt und auf 200 °C erhitzt. Innerhalb von 20 h bei 200 °C wurden ca. 18 ml Wasser abdestilliert. Dabei wurden ca. 426 g (Polypropylenglykol 600)-di(1-methyl-5-oxo-pyrrolidin-3-carboxylat) mit einer Verseifungszahl von 132 mg KOH/g erhalten.

### Beispiel 8

### Herstellung von Triethylenglykol-di(1-isobutyl-5-oxo-pyrrolidin-3-carboxylat)

In einem 500 ml Vierhalskolben mit Rührer, Thermometer, Stickstoffspülung sowie Destillationsbrücke wurden 185 g 1- Isobutyl-5-oxo-pyrrolidin-3-carbonsäure, 75 g Triethylenglykol sowie 1,5 g p-Toluolsulfonsäure vermischt und auf 200 °C erhitzt. Innerhalb von 8 h bei 200 °C wurden ca. 18 ml Wasser abdestilliert. Dabei wurden ca. 243 g Triethylenglykol-di(1-isobutyl-5-oxo-pyrrolidin-3-carboxylat) mit einer Verseifungszahl von 231 mg KOH/g erhalten.

### Beispiel 9

### Herstellung von (Trimethylolpropan + 9 EO)-tri(1-isobutyl-5-oxo-pyrrolidin-3-carboxylat)

In einem 500 ml Vierhalskolben mit Rührer, Thermometer, Stickstoffspülung sowie Destillationsbrücke wurden 185 g 1-Isobutyl -5-oxo-pyrrolidin-3-carbonsäure, 177 g Trimethylolpropan + 9 EO sowie 1,5 g p-Toluolsulfonsäure vermischt und auf 200 °C erhitzt. Innerhalb von 15 h bei 200 °C wurden ca. 18 ml Wasser abdestilliert. Dabei wurden ca. 346 g (Trimethylolpropan + 9 EO)-tri(1-isobutyl-5-oxo-pyrrolidin-3-carboxylat) mit einer Verseifungszahl von 163 mg KOH/g erhalten.

### Beispiel 10

### Herstellung von (Sorbitol + 18 EO)-tri(1-isobutyl-5-oxo-pyrrolidin-3-carboxylat)

In einem 1000 ml Vierhalskolben mit Rührer, Thermometer, Stickstoffspülung sowie Destillationsbrücke wurden 185 g 1-Isobutyl-5-oxo-pyrrolidin-3-carbonsäure, 325 g Sorbitol + 18 EO sowie 2,5 g p-Toluolsulfonsäure vermischt und auf 200 °C erhitzt. Innerhalb von 20 h bei 200 °C wurden ca. 18 ml Wasser abdestilliert. Dabei wurden ca. 492 g (Sorbitol + 18 EO)-tri(1-isobutyl-5-oxo-pyrrolidin-3-carboxylat) mit einer Verseifungszahl von 114 mg KOH/g erhalten.

### Beispiel 11

### Herstellung von (Sorbitol + 30 EO)-hexa(1-isobutyl-5-oxo-pyrrolidin-3-carboxylat)

In einem 1000 ml Vierhalskolben mit Rührer, Thermometer, Stickstoffspülung sowie Destillationsbrücke wurden 185 g 1-Isobutyl-5-oxo-pyrrolidin-3-carbonsäure, 250 g Sorbitol + 30 EO sowie 2,0 g p-Toluolsulfonsäure vermischt und auf 200 °C erhitzt. Innerhalb von 20 h bei 200 °C wurden ca. 18 ml Wasser abdestilliert. Dabei wurden ca. 419 g (Sorbitol + 30 EO)-hexa(1-isobutyl-5-oxo-pyrrolidin-3-carboxylat) mit einer Verseifungszahl von 135 mg KOH/g erhalten.

### Beispiel 12

### Herstellung von (Dekaglycerin + 30 EO)-poly(1-isobutyl-5-oxo-pyrrolidin-3-carboxylat)

In einem 1000 ml Vierhalskolben mit Rührer, Thermometer, Stickstoffspülung sowie Destillationsbrücke wurden 185 g 1-Isobutyl-5-oxo-pyrrolidin-3-carbonsäure, 390 g Dekaglycerin + 30 EO sowie 2,5 g p-Toluolsulfonsäure vermischt und auf 200 °C erhitzt. Innerhalb von 30 h bei 200 °C wurden ca. 18 ml Wasser abdestilliert. Dabei wurden ca. 556 g (Dekaglycerin + 30 EO)-poly(1-isobutyl-5-oxo-pyrrolidin-3-carboxylat) mit einer Verseifungszahl von 100 mg KOH/g erhalten.

### Referenz-Beispiel 13

### Herstellung von (Polypropylenglykol 400 + 10 EO)-di(1-isobutyl-5-oxo-pyrrolidin-3-carboxylat)

In einem 1000 ml Vierhalskolben mit Rührer, Thermometer, Stickstoffspülung sowie Destillationsbrücke wurden 185 g 1-Isobutyl-5-oxo-pyrrolidin-3-carbonsäure, 420 g Polypropylenglykol 400 + 10 EO sowie 2,5 g p-Toluoisulfonsäure vermischt und auf 200 °C erhitzt. Innerhalb von 18 h bei 200 °C wurden ca. 18 ml Wasser abdestilliert. Dabei wurden ca. 586 g (Polypropylenglykol 400 + 10 EO)-di(1-isobutyl-5-oxo-pyrrolidin-3-carboxylat) mit einer Verseifungszahl von 95 mg KOH/g erhalten.

### Referenz-Beispiel 14

### Herstellung von (Polypropylenglykol 600)-di(1-isobutyl-5-oxo-pyrrolidin-3-carboxylat)

In einem 1000 ml Vierhalskolben mit Rührer, Thermometer, Stickstoffspülung sowie Destillationsbrücke wurden 185 g 1-Isobutyl-5-oxo-pyrrolidin-3-carbonsäure, 300 g Polypropylenglykol 600 sowie 2,0 g p-Toluolsulfonsäure vermischt und auf 200 °C erhitzt. Innerhalb von 20 h bei 200 °C wurden ca. 18 ml Wasser abdestilliert. Dabei wurden ca. 467 g (Polypropylenglykol 600)-di(1-isobutyl-5-oxo-pyrrolidin-3-carboxylat) mit einer Verseifungszahl von 120 mg KOH/g erhalten.

### Beispiel 15

### Herstellung von Triethylenglykol-di(1-oleyl-5-oxo-pyrrolidin-3-carboxylat)

In einem 1000 ml Vierhalskolben mit Rührer, Thermometer, Stickstoffspülung sowie Destillationsbrücke wurden 380 g 1-Oleyl-5-oxo-pyrrolidin-3-carbonsäure, 75 g Triethylenglykol sowie 2,0 g p-Toluolsulfonsäure vermischt und auf 200 °C erhitzt. Innerhalb von 8 h bei 200 °C wurden ca. 18 ml Wasser abdestilliert. Dabei wurden ca. 438 g Triethylenglykol-di(1-oleyl-5-oxo-pyrrolidin-3-carboxylat) mit einer Verseifungszahl von 129 mg KOH/g erhalten.

### Beispiel 16

### Herstellung von (Trimethylolpropan + 9 EO)-tri(1-oleyl-5-oxo-pyrrolidin-3-carboxylat)

In einem 1000 ml Vierhalskolben mit Rührer, Thermometer, Stickstoffspülung sowie Destillationsbrücke wurden 380 g 1-Oleyl -5-oxo-pyrrolidin-3-carbonsäure, 177 g Trimethylolpropan + 9 EO sowie 2,5 g p-Toluolsulfonsäure vermischt und auf 200 °C erhitzt. Innerhalb von 15 h bei 200 °C wurden ca. 18 ml Wasser abdestilliert. Dabei wurden ca. 540 g (Trimethylolpropan + 9 EO)-tri(1-oleyl-5-oxo-pyrrolidin-3-carboxylat) mit einer Verseifungszahl von 105 mg KOH/g erhalten.

### Beispiel 17

### Herstellung von (Sorbitol + 18 EO)-tri(1-oleyl-5-oxo-pyrrolidin-3-carboxylat)

In einem 1000 ml Vierhalskolben mit Rührer, Thermometer, Stickstoffspülung sowie Destillationsbrücke wurden 380 g 1-Oleyl-5-oxo-pyrrolidin-3-carbonsäure, 325 g Sorbitol + 18 EO sowie 3,0 g p-Toluolsulfonsäure vermischt und auf 200 °C erhitzt. Innerhalb von 20 h bei 200 °C wurden ca. 18 ml Wasser abdestilliert. Dabei wurden ca. 686 g (Sorbitol + 18 EO)-tri(1-oleyl-5-oxo-pyrrolidin-3-carboxylat) mit einer Verseifungszahl von 82 mg KOH/g erhalten.

### Beispiel 18

### Herstellung von (Sorbitol + 30 EO)-hexa(1-oleyl-5-oxo-pyrrolidin-3-carboxylat)

In einem 1000 ml Vierhalskolben mit Rührer, Thermometer, Stickstoffspülung sowie Destillationsbrücke wurden 380 g 1-Oleyl-5-oxo-pyrrolidin-3-carbonsäure, 250 g Sorbitol + 30 EO sowie 3,0 g p-Toluolsulfonsäure vermischt und auf 200 °C erhitzt. Innerhalb von 20 h bei 200 °C wurden ca. 18 ml Wasser abdestilliert. Dabei wurden ca. 613 g (Sorbitol + 30 EO)-hexa(1-oleyl-5-oxo-pyrrolidin-3-carboxylat) mit einer Verseifungszahl von 92 mg KOH/g erhalten.

### Beispiel 19

### Herstellung von (Dekaglycerin + 30 EO)-poly(1-oleyl-5-oxo-pyrrolidin-3-carboxylat)

In einem 1000 ml Vierhalskolben mit Rührer, Thermometer, Stickstoffspülung sowie Destillationsbrücke wurden 380 g 1-Oleyl-5-oxo-pyrrolidin-3-carbonsäure, 390 g Dekaglycerin + 30 EO sowie 3,0 g p-Toluolsulfonsäure vermischt und auf 200 °C erhitzt. Innerhalb von 30 h bei 200 °C wurden ca. 18 ml Wasser abdestilliert. Dabei wurden ca. 750 g (Dekaglycerin + 30 EO)-poly(1-oleyl-5-oxo-pyrrolidin-3-carboxylat) mit einer Verseifungszahl von 72 mg KOH/g erhalten.

### Referenz-Beispiel 20

### Herstellung von (Polypropylenglykol 400 + 10 EO)-di(1-oleyl-5-oxo-pyrrolidin-3-carboxylat)

In einem 1000 ml Vierhalskolben mit Rührer, Thermometer, Stickstoffspülung sowie Destillationsbrücke wurden 380 g 1-Oleyl-5-oxo-pyrrolidin-3-carbonsäure, 420 g Polypropylenglykol 400 + 10 EO sowie 3,0 g p-Toluolsulfonsäure vermischt und auf 200 °C erhitzt. Innerhalb von 18 h bei 200 °C wurden ca. 18 ml Wasser abdestilliert. Dabei wurden ca. 780 g (Polypropylenglykol 400 + 10 EO)-di(1-oleyl-5-oxo-pyrrolidin-3-carboxylat) mit einer Verseifungszahl von 72 mg KOH/g erhalten.

### Referenz-Beispiel 21

### Herstellung von (Polypropylenglykol 600)-di(1-oleyl-5-oxo-pyrrolidin-3-carboxylat)

In einem 1000 ml Vierhalskolben mit Rührer, Thermometer, Stickstoffspülung sowie Destillationsbrücke wurden 380 g 1-Oleyl-5-oxo-pyrrolidin-3-carbonsäure, 300 g Polypropylenglykol 600 sowie 3,0 g p-Toluolsulfonsäure vermischt und auf 200 °C erhitzt. Innerhalb von 20 h bei 200 °C wurden ca. 18 ml Wasser abdestilliert. Dabei wurden ca. 660 g (Polypropylenglykol 600)-di(1-oleyl-5-oxo-pyrrolidin-3-carboxylat) mit einer Verseifungszahl von 85 mg KOH/g erhalten.

### Wirksamkeit der erfindungsgemäßen Verbindungen als Gashydratinhibitoren

Zur Untersuchung der inhibierenden Wirkung der erfindungsgemäßen Verbindungen wurde ein Stahlrührautoklav mit Temperatursteuerung, Druck- und Drehmomentaufnehmer mit 450 ml Innenvolumen benutzt. Für Untersuchungen zur kinetischen Inhibierung wurde der Autoklav mit destillierten Wasser und Gas im Volumenverhältnis 20:80 gefüllt, für Untersuchungen der Agglomeratinhibierung wurde zusätzlich Kondensat zugegeben. Anschließend wurden 50 bar Erdgas aufgedrückt.

Ausgehend von einer Anfangstemperatur von 20°C wurde innerhalb 3 h auf 4 °C abgekühlt, dann 18 h bei 4 °C gerührt und innerhalb von 2 h wieder auf 20 °C aufgeheizt. Dabei wird zunächst eine Druckabnahme gemäß der thermischen Kompression des Gases beobachtet. Tritt während der Unterkühlzeit die Bildung von Gashydratkeimen auf, so verringert sich der gemessene Druck, wobei ein Anstieg des gemessenen Drehmoments und ein leichter Anstieg der Temperatur zu beobachten ist. Weiteres Wachstum und zunehmende Agglomeration der Hydratkeime führt ohne Inhibitor schnell zu einem weiteren Anstieg des Drehmomentes. Beim Aufwärmen des Gemisches zerfallen die Gashydrate, so dass man den Anfangszustand der Versuchsreihe erreicht.

Als Maß für die inhibierende Wirkung der erfindungsgemäßen Verbindungen wird die Zeit vom Erreichen der Minimaltemperatur von 4 °C bis zur ersten Gasaufnahme (T_{ind}) bzw. die Zeit bis zum Anstieg des Drehmomentes (T_{agg}) benutzt. Lange Induktionszeiten bzw. Agglomerationszeiten weisen auf eine Wirkung als kinetischer Inhibitor hin. Das im Autoklaven gemessene Drehmoment dient dagegen als Größe für die Agglomeration der Hydratkristalle. Bei einem guten Antiagglomerant ist das Drehmoment, das sich nach Bildung von Gashydraten aufbaut, gegenüber dem Blindwert deutlich verringert. Im Idealfall bilden sich schneeartige, fein verteilte Hydratkristalle in der Kondensatphase, die sich nicht zusammenlagern und somit nicht zum Verstopfen der zum Gastransport und zur Gasförderung dienenden Installationen führen.

### Testergebnisse

Zusammensetzung des verwendeten Erdgases:
Methan 84,8 %, Ethan 9,2 %, Propan 2,6 %, Butan 0,9 %, Kohlendioxid 1,6 %,
Stickstoff 0,9 %.

Als Vergleichssubstanz wurde ein kommerziell erhältlicher Gashydratinhibitor auf Basis Polyvinylpyrrolidon verwendet. Die Dosierrate betrug bei allen Versuchen 5000 ppm bezogen auf die Wasserphase.

| 1-Alkyl-5-oxo-pyrrolidin-3-carbonsäureester aus Beispiel | T_{ind} (h) | T_{agg} (h) |
|---|---|---|
| Blindwert | 0 | 0 |
| 1 | 12,4 | 12,5 |
| 2 | 13,1 | 13,3 |
| 3 | 14,1 | 14,1 |
| 4 | 14,3 | 14,5 |
| 5 | 12,8 | 13,0 |
| 6 | 9,9 | 10,1 |
| 8 | 20,9 | 22,1 |
| 9 | 22,0 | 23,4 |
| 10 | 24,0 | 24,9 |
| 11 | 25,7 | 26,5 |
| 12 | 23,3 | 23,5 |
| 13 | 15,1 | 15,8 |
| Vergleich | 3,5 | 3,6 |

Wie aus den obigen Testresultaten zu erkennen ist, wirken die erfindungsgemäßen 1-Alkyl-5-oxo-pyrrolidin-3-carbonsäureester als kinetische Gashydratinhibitoren und zeigen eine deutliche Verbesserung gegenüber dem Stand der Technik.

Um die Wirkung als Agglomeratinhibitoren zu prüfen, wurde im oben verwendeten Testautoklaven Wasser und Testbenzin vorgelegt (20 % des Volumens im Verhältnis 1:2) und bezogen auf die Wasserphase 5000 ppm des jeweiligen Additivs zugegeben.
Bei einem Autoklavendruck von 50 bar und einer Rührgeschwindigkeit von 500 U/min wurde die Temperatur von anfangs 20 °C innerhalb 3 Stunden auf 4 °C abgekühlt, dann 18 Stunden bei 2 °C gerührt und wieder aufgewärmt. Dabei wurden die Agglomerationszeit bis zum Auftreten von Gashydrat-Agglomeraten und das dabei auftretende Drehmoment am Rührer gemessen, das ein Maß für die Agglomeration der Gashydrate ist.

Als Vergleichssubstanz wurde ein kommerziell erhältlicher Anti-Agglomerant (quartäres Ammoniumsalz) herangezogen.

| 1-Alkyl-5-oxo-pyrrolidin-3-carbonsäureester aus Beispiel | T_{agg} (h) | Mₘₐₓ (Ncm) |
|---|---|---|
| Blindwert | 0,1 | 15,9 |
| 6 | 7,5 | 0,9 |
| 7 | 6,9 | 1,0 |
| 13 | 8,9 | 1,0 |
| 14 | 10,5 | 1,0 |
| 15 | 4,1 | 1,6 |
| 16 | 4,8 | 1,8 |
| 17 | 4,5 | 1,5 |
| 18 | 4,5 | 2,0 |
| 19 | 4,6 | 2,1 |
| 20 | 4,9 | 1,6 |
| 21 | 4,0 | 1,7 |
| Vergleich | 2,6 | 4,1 |

Wie aus diesen Beispielen zu ersehen ist, sind die gemessenen Drehmomente im Vergleich zum Blindwert trotz Gashydratbildung stark reduziert. Dies spricht für eine deutliche agglomerat-inhibierende Wirkung der erfindungsgemäßen Verbindungen. Zusätzlich weisen die erfindungsgemäßen Verbindungen unter den Versuchsbedingungen auch eine deutliche Wirkung als kinetische Inhibitoren auf. Sämtliche Beispiele zeigen eine deutlich bessere Performance als der kommerziell erhältliche Anti-Agglomerant (Vergleich = Stand der Technik).

Im Folgenden wird die deutlich verbesserte biologische Abbaubarkeit (nach OECD 306) der erfindungsgemäßen Verbindungen im Vergleich zum Stand der Technik (kommerziell erhältliches Polyvinylpyrrolidon) aufgezeigt.

| 1-Alkyl-5-oxo-pyrrolidin-3-carbonsäureester aus Beispiel | Biologische Abbaubarkeit 28 Tage (OECD 306) |
|---|---|
| Polyvinylpyrrolidon | 5 |
| 1 | 65 |
| 2 | 61 |
| 3 | 70 |
| 4 | 72 |
| 5 | 45 |
| 6 | 30 |
| 7 | 25 |
| 8 | 69 |
| 9 | 66 |
| 10 | 73 |
| 11 | 73 |
| 12 | 38 |
| 13 | 33 |
| 14 | 25 |
| 15 | 42 |
| 16 | 40 |
| 17 | 36 |
| 18 | 35 |
| 19 | 33 |
| 20 | 23 |
| 21 | 24 |

## Patentansprüche

1. Verbindungen der Formel 1 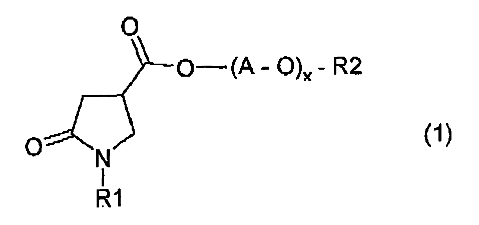 worin
A eine C₂- bis C₄-Alkylengruppe
x eine Zahl von 1 bis 100,
y eine Zahl von 0 bis 100 bedeuten,
R1 für C₁-C₃₀-Alkyl, C₁-C₃₀-Alkenyl, C₇-C₃₀-Alkylaryl steht,
R2 für einen organischen Rest der Formel 3 steht 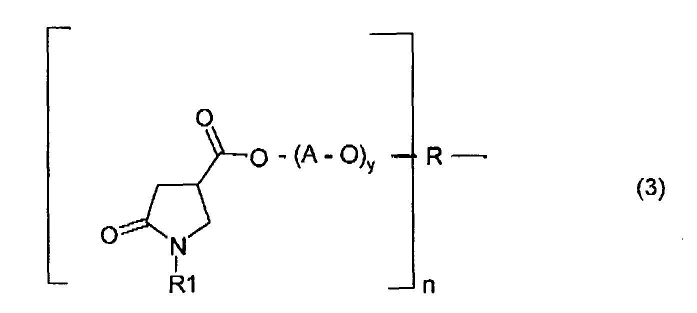 worin n für eine Zahl von 1 bis 100 steht, und R entweder
(1) durch formale Abstraktion der Wasserstoffatome der OH-Gruppen von Ethylenglykol, Propylenglykol, Glycerin, Diglycerin, Triglycerin, Tetraglycerin, Polyglycerin, Trimethylolpropan, Pentaerythrit, Dipentaerythrit, Tripentaerythrit, Sorbitol, Sorbitan, Diethylenglykol, Triethylenglykol, Dipropylenglykol, Tripropylenglykol und Tris(hydroxymethyl)aminomethan gebildet wird,
oder
(2) für einen Alkylen- oder Polyalkylenoxid-Rest mit 1 bis 30 Kohlenstoffatomen steht.

2. Verbindungen der Formel 4 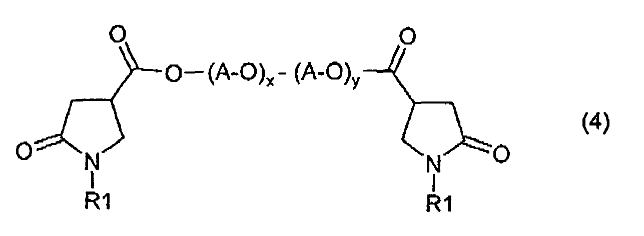 worin
A eine C₂- bis C₄-Alkylengruppe
x eine Zahl von 1 bis 100,
y eine Zahl von 1 bis 100 bedeuten, und
R1 für C₁-C₃₀-Alkyl, C₂-C₃₀-Alkenyl, C₇-C₃₀-Alkylaryl steht.

3. Verbindungen gemäß Anspruch 1 oder 2, wobei x und y unabhängig voneinander eine Zahl von 2 bis 80 bedeuten.

4. Verbindungen gemäß einem oder mehreren der Ansprüche 1 bis 3, wobei A für Ethylenreste, Propylenreste oder Mischungen aus Ethylen- und Propylenresten steht.

5. Verwendung von Verbindungen gemäß einem oder mehreren der Ansprüche 1 bis 4 in Mengen von 0,01 bis 2 Gew.-% (bezogen auf das Gewicht der wässrigen Phase) zur Verhinderung der Bildung von Gashydraten in wässrigen Phasen, die mit einer gasförmigen, flüssigen oder festen organischen Phase in Verbindung stehen.

## Claims

1. A compound of the formula 1 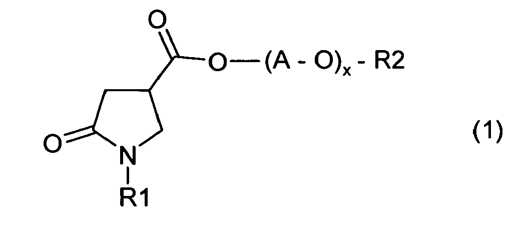 in which
A is a C₂- to C₄-alkylene group
x is from 1 to 100,
y is from 0 to 100,
R1 is C₁-C₃₀-alkyl, C₂-C₃₀-alkenyl, C₇-C₃₀-alkylaryl,
R2 is an organic radical of the formula 3 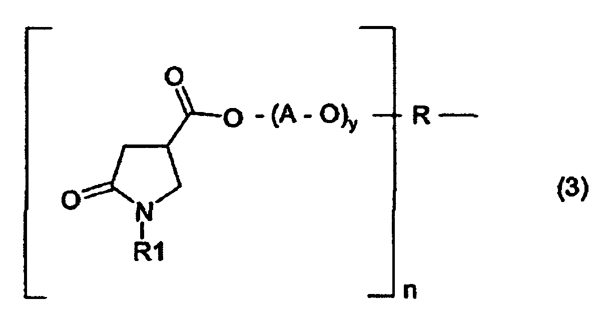 in which n is from 1 to 100 and R is either
(1) formed through formal abstraction of the hydrogen atoms of the OH groups of ethylene glycol, propylene glycol, glycerol, diglycerol, triglycerol, tetraglycerol, polyglycerol, trimethylolpropane, pentaerythritol, dipentaerythritol, tripentaerythritol, sorbitol, sorbitan, diethylene glycol, triethylene glycol, dipropylene glycol, tripropylene glycol and tris(hydroxymethyl)aminomethane,
or
(2) an alkylene or polyalkylene oxide radical having from 1 to 30 carbon atoms.

2. A compound of the formula 4 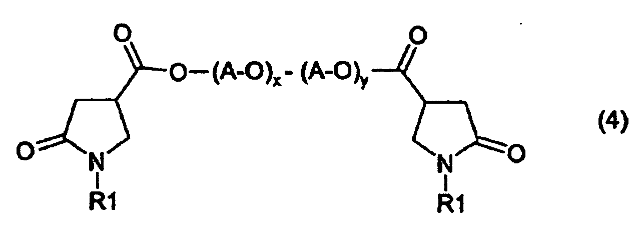 in which
A is a C₂- to C₄-alkylene group
x is from 1 to 100,
y is from 1 to 100,
R1 is C₁-C₃₀-alkyl, C₂-C₃₀-alkenyl, C₇-C₃₀-alkylaryl.

3. A compound as claimed in claim 1 or 2, wherein x and y are each independently from 2 to 80.

4. A compound as claimed in one or more of claims 1 to 3, wherein A represents ethylene radicals, propylene radicals or mixtures of ethylene and propylene radicals.

5. The use of compounds as claimed in one or more of claims 1 to 4 in amounts of from 0.01 to 2% by weight (based on the weight of the aqueous phase) for preventing the formation of gas hydrates in aqueous phases which are in contact with a gaseous, liquid or solid organic phase.

## Revendications

1. Composés de formule 1 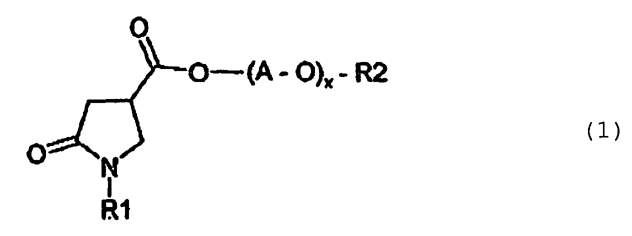 dans laquelle
A représente un groupe alkylène en C₂-C₄
x représente un nombre valant de 1 à 100,
y représente un nombre valant de 0 à 100,
R1 représente un groupe alkyle en C₁-C₃₀, alcényle en C₂-C₃₀, alkylaryle en C₇-C₃₀,
R2 représente un radical organique de formule 3 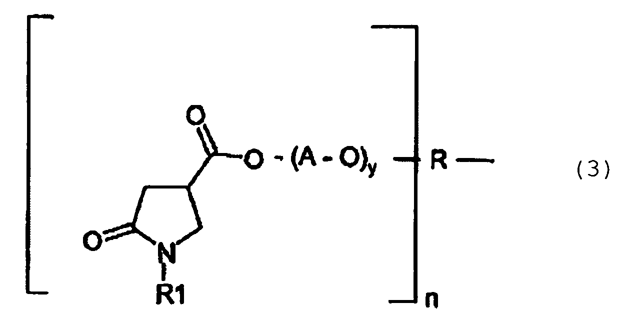 dans laquelle n représente un nombre valant de 1 à 100, et R soit
(1) est constitué par élimination des atomes d'hydrogène des groupes OH d'éthylèneglycol, de propylèneglycol, glycérol, diglycérol, triglycérol, tétraglycérol, polyglycérol, triméthylolpropane, pentaérythritol, dipentaérythritol, tripentaérythritol, sorbitol, sorbitanne, diéthylèneglycol, triéthylèneglycol, dipropylèneglycol, tripropylène- glycol et tris(hydroxyméthyl)aminométhane,
OU
(2) représente un radical oxyalkylène ou polyoxyalkylène ayant de 1 à 30 atomes de carbone.

2. Composés de formule 4 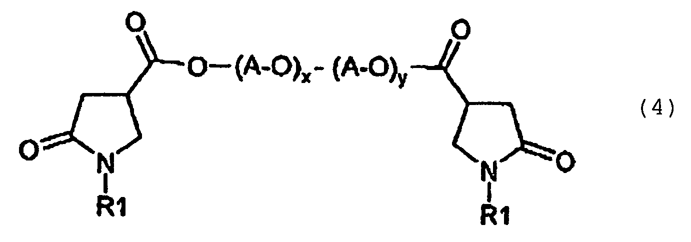 dans laquelle
A représente un groupe alkylène en C₂-C₄
x représente un nombre valant de 1 à 100,
y représente un nombre valant de 1 à 100, et
R1 représente un groupe alkyle en C₁-C₃₀, alcényle en C₂-C₃₀, alkylaryle en C₇-C₃₀.

3. Composés selon la revendication 1 ou 2, dans lesquels x et y représentent, indépendamment l'un de l'autre, un nombre valant de 2 à 80.

4. Composés selon une ou plusieurs des revendications 1 à 3, dans lesquels A représente des radicaux éthylène, des radicaux propylène ou des mélanges de radicaux éthylène et propylène.

5. Utilisation des composés selon une ou plusieurs des revendications 1 à 4, en quantités de 0,01 à 2 % en poids (par rapport au poids de la phase aqueuse), pour empêcher la formation d'hydrates de gaz dans des phases aqueuses qui sont en communication avec une phase organique gazeuse, liquide ou solide.
